# EUROPEAN PATENT SPECIFICATION

(11) **EP 3 041 510 B1**
(45) Date of publication and mention of the grant of the patent: **08.01.2020**
(21) Application number: 14762112.2
(22) Date of filing: 25.07.2014
(51) Int. Cl.: A61K 47/34, A61K 41/00, A61K 9/107

(54) **POLYMER MICELLE CONTAINING A PHOTOSENSITIZER**
POLYMERE MIZELLEN ENTHALTEND EIN PHOTOSENSIBILISATOR
MICELLES POLYMÈRES CONTENANT UN PHOTOSENSIBILISATEUR

(30) Priority: 04.09.2013 PL 40524113
(43) Date of publication of application: 13.07.2016
(73) Proprietor: Siec Badawcza Lukasiewicz - PORT Polski Osrodek Rozwoju Technologii, 54-066 Wroclaw (PL)
(72) Inventor: FRACKOWIAK, Renata, 51-211 Wroclaw (PL); CIBOR, Urszula, PL-59-600 Lwówek Slaski (PL); WILK, Kazimiera, 51-361 Wilczyce (PL); GAMIAN, Andrzej, PL-51-644 Wroclaw (PL)
(74) Representative: Godlewski, Piotr
(86) International application number: PCT/PL2014/050046
(87) International publication number: WO 2015/034381

(56) References cited:
- US-A1- 2010 129 432
- US-A1- 2011 085 987
- US-A1- 2012 225 017
- RUBINAH K. CHOWDHARY ET AL: "Formulation of Benzoporphyrin Derivatives in Pluronics", PHOTOCHEMISTRY AND PHOTOBIOLOGY, vol. 77, no. 3, 1 March 2003 (2003-03-01), pages 299-303, XP055052858, ISSN: 0031-8655, DOI: 10.1562/0031-8655(2003)077<0299:FOBDIP>2.0 .CO;2
- CHENG-LIANG PENG ET AL: "Multimodal Image-Guided Photothermal Therapy Mediated by 188 Re-Labeled Micelles Containing a Cyanine-Type Photosensitizer", ACS NANO, vol. 5, no. 7, 26 July 2011 (2011-07-26), pages 5594-5607, XP055148510, ISSN: 1936-0851, DOI: 10.1021/nn201100m
- BOEHM TOBIAS K ET AL: "Diode laser activated indocyanine green selectively kills bacteria", JOURNAL OF THE INTERNATIONAL ACADEMY OF PERIODONTOLOGY, FDI WORLD DENTAL PRESS, LONDON, GB, vol. 13, no. 2, 1 July 2011 (2011-07-01), pages 58-63, XP008153251, ISSN: 1466-2094
- DATABASE WPI Week 201220 Thomson Scientific, London, GB; AN 2012-B51059 XP002734881, & KR 2012 0007392 A (KOREA INST SCI&TECHNOLOGY) 20 January 2012 (2012-01-20)
- PIETKIEWICZ J ET AL: "New approach to hydrophobic cyanine-type photosensitizer delivery using polymeric oil-cored nanocarriers: Hemolytic activity, in vitro cytotoxicity and localization in cancer cells", EUROPEAN JOURNAL OF PHARMACEUTICAL SCIENCES, ELSEVIER, AMSTERDAM, NL, vol. 39, no. 5, 18 March 2010 (2010-03-18) , pages 322-335, XP026935210, ISSN: 0928-0987, DOI: 10.1016/J.EJPS.2009.12.012 [retrieved on 2010-01-07]
- FENG JIANG ET AL: "Photodynamic Therapy of 9L Gliosarcoma with Liposome-Delivered Photofrin", PHOTOCHEMISTRY AND PHOTOBIOLOGY, vol. 65, no. 4, 1 April 1997 (1997-04-01), pages 701-706, XP055163416, ISSN: 0031-8655, DOI: 10.1111/j.1751-1097.1997.tb01913.x
- Timothy J Kinsella ET AL: "Photodynamic therapy in oncology", EXPERT OPINION ON PHARMACOTHERAPY, vol. 2, no. 6, 1 June 2001 (2001-06-01), pages 917-927, XP055482587, LONDON, UK ISSN: 1465-6566, DOI: 10.1517/14656566.2.6.917

## Description

The present invention relates to the polymer micelle nanocarriers based on block copolymers with the trade name Pluronic produced using thin film hydration, as active ingredient carriers of the photosensitizer type for use in photodynamic therapy of tumours and a method of producing the polymer micelle.

In light of the increasing incidence of age-related diseases, such as neoplastic diseases, the construction of drug carriers and active ingredient transport systems is currently one of the main goals of the pharmaceutical industry. The design and production of novel drugs and therapies is time and labour intensive. Therefore, a large opportunity for finding new solutions lays in the modification and perfection of extant therapies. One such approach is the so called targeted therapy which entails the use of drug nanocarriers which facilitate the delivery of an active ingredient to a particular site and the reduction of its side effects in the system [R.C. Pinto et al. Nanomedicine: Nanotechnology, Biology, and Medicine 2 (2006) 8]. A polymer micelle, due to its structure and relatively simple production process, is a very promising carrier, which may be useful in the solubilisation and effective delivery of many drugs into tumour cells [N. Rapoport Progress in Polymer Science 32 (2007) 962]. They differ from other drug carriers by their small size, extensive transparency of solutions, an internal hydrophobic phase, and protective activity for the external polymer chains. Polymer micelles may primarily be carriers for hydrophobic and poorly soluble drugs [U. Kedar et al. Nanomedicine: Nanotechnology, Biology, and Medicine 6 (2010) 714], but there is also a possibility of solubilizing combinations of hydrophilic substances along the palisade-like structure of the hydrophilic crown [X. Y. Xionga et al. Journal of Controlled Release 103 (2005) 73]. A key parameter that characterises micelle formation is the micelle critical concentration (CMC). At CMC and slightly above this concentration, polymer micelles may be characterised by a relatively loose structure and the presence of water molecules in the core. However, along with an increase in the concentration, copolymer micelles become more dense, stable and slightly smaller due to the exclusion of water molecules from their cores. The lower the CMC for a given copolymer, the more stable are the micelles formed. This is particularly important from a pharmaceutical standpoint, as when they are diluted in a large volume of blood, only micelles with a low CMC value can still exist in aggregated form. Micelles with a high CMC may degrade, releasing the solubilised drug into healthy blood vessels, which in consequence may result in the lack of a therapeutic effect, or else a toxic effect in healthy tissues [V. P. Torchilin, Journal of Controlled Release 73 (2001) 137]. Micelles as a whole should not have diameters larger than 100 nm so as to avoid reticuloendothelial system reactions (RES) [F. Danhier et al. Journal of Controlled Release 148 (2010) 135; H. Liua et al. Journal of Controlled Release 68 (2000) 167]. At the same time, due to their small size, it should facilitate cell and tissue entry, but should not be smaller than 10nm [N. Rapoport, Progress in Polymer Science 32 (2007) 962]. Another requirement is also the low polydispersity of solutions due to unpredictable changes in pharmacokinetic parameters of the drug and its distribution in organs, potentially caused by highly polydispersive micelle solutions. Polymer micelles must retain their stability under highly diluted conditions that occur during injection. The micelle stern layer should be an effective spherical barrier and tightly surround the core to ensure the isolation of the solubilised drug from the environment. The micelle core should have a high loading capacity and allow for the controlled release of the drug.

Cyanins as potential photosensitizers w photodynamic therapy have been studied for over two decades. Various chemical structures may possess groups of varying hydrophobicity and electrical charges, in effect absorbing light at varying wavelengths. Cyanins usually undergo excitation at wavelengths above 500 nm, wherein this feature is characteristic for a given cyanin. In most cases, this range is 600-1000 nm, in the "phototherapeutic window". At these wavelengths, light dispersion is low in tissues, and at the same time penetrance grows. The biggest advantages of cyanins is their effectiveness as photosensitizers and their many forms, which are for the most part commercially available [E. Delaey et al. Journal of Photochemistry and Photobiology B: Biology 55 (2000) 27]. However, their hydrophobicity and, therefore, their insolubility in water, and their sensitivity to light and temperature precludes their direct use in photodynamic therapy. It is therefore justified to create carrier systems for them which increase their solubility and make it possible to selectively supply them to the interior of tumour cells at the same time protecting them from premature degradation in the human organism.

American patent description US7014839B2 discloses a method of mixing for the manufacture phototagged polymer micelles based on Pluronic F108 loaded with indocyanin (ICG) and meant for use as cell markers for *in vivo* diagnostic light imaging.

American patent description US7356368B2 discloses medical products consisting of polymer coatings made of triblock copolymers: Pluronic 105 or Pluronic 127, containing A,A-diethylacrylamide (as a stabilizer for the hydrophobic micelle interior) and a medicinal product encapsulated in the micelle. Micelles are dried and used directly as medicinal product coatings.

American patent description US2010/0131001A1 discloses systems and methods of treating aneurysms using polymer micelles containing a medicinal product released when acted upon by light, ultrasound, radio, UV, IR or visible light energy or a combination thereof.

American patent description US2010/0310660 makes use of polymer micelle-type nanostructures containing a medicinal product, meant for use in inhalers for delivering an active ingredient (heparin) into the lungs. Polymer micelles were manufactured using the thin film method using block copolymers: Pluronic L122, Pluronic P105 and Pluronic F127.

Polish patent description PL202475 discloses a composition forming micelles, containing a therapeutic agent enclosed in the micelle, characterised in that the hydrophobic core is selected from a group encompassing polyorthoester, polyanhydrate, pseudopoly(aminoacid) derived from tyrosine, polyphosphoazene or poly(P-benzyl-L-aspartate) and their combinations, or a polyester selected from a group encompassing poly(glycolic acid), poly(lactic acid), poly(D-lactic acid), poly(D,L-lactic acid), the copolymers of lactide/glycolide or polycaprolactone, and the hydrophilic coating is poly(A-vinyl-2- pyrolidone).

American patent description US2011/0142951A1 makes use of biocompatible, stable, non-toxic polymer micelles made based on block copolymers, containing a therapeutic agent such as a nucleotide or peptide. The polymer micelle produced protect the therapeutic agent against degradation, deliver it into cells via a natural process (endocytosis) and/or during contact with cells deliver the therapeutic agent into the cytoplasm.

American patent description US2011/0223206A1 discloses a method of making block copolymers using, i.e. PLGA, PLA, PCL, PEG and polymer micelles made based on the synthesized block copolymers. Nanostructures according to that invention are useful as carriers of antitumours, anti-inflammatory, antimycotic, and antihypertension drugs as well as hormones and steroids.

American patent descriptions US2011/0224151A1 and US2011/0251269A1 disclose polymer micelles made of di-block copolymers (mPEG-PLA), containing solubilised drugs such as Paclitaxel, Cyclosporin and Docetaxel, designed for anti-cancer therapy.

International application publication WO2011/113616A1 discloses intelligent polymer micelles containing an active ingredient and a contrast agent for MRI imaging designed for the early detection of breast cancer. The polymer micelles were produced from copolymers selected from among: polyethylene glycol-b-trimethylsilyl methacrylate, PEG-b-pHis/PLA, PLGA-b-PEG-COOH.

R.K. Chowdhary et al. (Photochemistry and Photobiology, 2003, 77(3): 299-303) describes potential of Pluronics for the formulation of tetrapyrrole-based photosensitizers, with a particular focus on B-ring benzoporphyrin derivatives. The best performance was observed in the micelle-forming Pluronic P123 and L122 due to their optimal length of hydrophobic block resulting in a better stabilization of tetrapyrrolic drugs in monomeric form in aqueous suspensions.

Cheng-Liang Peng et al. (ACS NANO, vol.5, no.7, p.5594-5607, 2011, DOI: 10.1021/nn201100m) describes micellar systems comprising IR-780 iodide photosensitiser, for use in photothermal therapy (PTT) of cancer. However, the micelles comprise different copolymers, i.e.: mPEG-b-PCL and DTPA-PEG-b-PCL copolymers. The resulting micellar systems may demonstrate encapsulation efficiency even of 93.8%, but at the same time PdI is relatively high of about 0.3.

The subject of the present invention therefore is a polymer micelle containing an active ingredient for use in photodynamic therapy of tumours, wherein said micelle, produced using hydration method of a thin film of block copolymers, contains block copolymers: poly(ethylene oxide-&-propylene oxide-&-ethylene oxide) with the general formula 1, 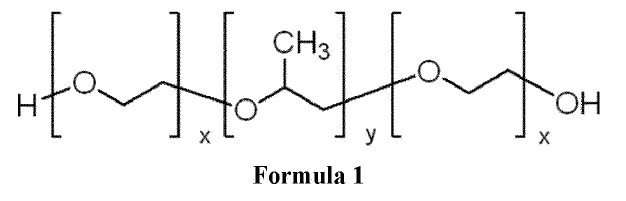 in which
x denotes the number of polyoxyethylene groups, with a value from 5 to 100 ;
y denotes the number of polyoxypropylene groups, with a value from 65 to 69; whereas the active ingredient is selected from a group of photosensitizers encompassing cyanins
wherein said block copolymer is a copolymer (PEO)₂₀-(PPO)₆₉-(PEO)₂₀ (Pluronic P123) and
said active ingredient is 2-[2-[2-chloro-3-[(1,3-dihydro-3,3-dimethyl-1-propyl-2H-indolyl-2-ylidenyl)ethylidenyl]-1-cyclohexen-1-yl]ethenyl]-3,3-dimethyl-1-propylindole iodide (cyanin IR-780).

The next subject of the present invention is a method of producing a polymer micelle according to the present invention, characterised in that block copolymers previously dissolved in a volatile organic solvent and an active ingredient are mixed, and then the solvent is evaporated off on a rotating evaporator and the thin film formed is hydrated, wherein a solution of the copolymers is prepared with a concentration of at least a hundredfold greater than its critical micelle concentration and is mixed with a photosensitizer also dissolved in an organic solvent with a concentration of 0.01-0.2 g/l, and then the solvent is evaporated off on a rotating evaporator, resulting in thin film, which is then hydrated at a temperature of 25-60°C for 0.5-2h and an aqueous solution of micelles is obtained with the photosensitizer solubilized inside the micelle,
wherein said block copolymer is the copolymer (PEO)₂₀-(PPO)₆₉- (PEO)₂₀, the active ingredient is 2-[2-[2-chloro-3-[(1,3-dihydro-3,3-dimethyl-1-propyl-2H-indolyl-2-ylidenyl)ethylidenyl]-1-cyclohexen-1-yl]ethenyl]-3,3-dimethyl-1-propylindole iodide (cyanin IR-780),
and the organic solvent used is selected from a group encompassing: tetrahydrofuran, methanol, ethanol, isopropanol, chloroform, more preferably methanol.

Preferably, the photosensitizer solution used in the method according to the present invention is prepared at a concentration of 0.04-0.16 g/l.

Preferably, in the method according to the present invention, the thin film hydration is performed at a temperature of 40-50°C, for 1 hour.

In order to remove the unsolubilised dye, in the method according to the present invention, micelle solutions preferably are filtered through syringe filters, preferably through filters with a pore diameter of 0.2µm.

The crux of the present invention are new polymer carriers of the polymer micelle type based on P123 for photosensitizer IR-780 selected from among the group encompassing cyanins, obtained via thin film hydration using block copolymers with the trade name Pluronic with the general formula 1.

The advantage of a polymer micelle according to the present invention, comprising P123 copolymer and cyanine IR-780 as active ingredient, is its unexpected combination of preferable properties i.e.: very low polydispersity (PdI =0.058) and nanoscopic size (<20 nm) and a very high degree of solubilisation of 86.5%, ideal for use as drug carriers in antitumours therapy. Additionally, they exhibit potential anti-tumour activity for use in photodynamic therapy, through their low cytotoxicity and increased photocytotoxicity against the model cell line MCF-7.

A definite advantage of the present invention is the production via a simple method of stable nanocarriers for active ingredients based on safe, non-toxic, variable and commercially available block copolymers such as Pluronic, biocompatible with many hydrophobic drugs. The low cost of Pluronic polymers, literature-confirmed efficacy in the solubilisation of many various hydrophobic compounds and a large structural variety makes the production of polymer micelles on the basis of these compounds simpler and much less expensive. Furthermore, the cytotoxicity and photocytotoxicity tests performed on polymer micelles loaded with photosensitizers confirmed the capability of these systems to penetrate tumour cells which warrants their potential for use in photodynamic therapy, as well as in other therapies where it is required to deliver a given compound into the interior of a tumour cell.

The subject of the present invention is explained in example embodiments of a polymer micelle based on Pluronic-type block copolymer, P123, loaded with photosensitizer cyanin IR-780. Additionally, examples of other micellar systems based on Pluronic-type block copolymers such as P123, F127, L121 loaded with photosensitizers such as cyanins and porphyrins selected from: 2-(2-[2-chloro-3-([1,3-dihydro-1,3,3-trimethyl-2H-indolyl-2-ylidenyl]ethylidenyl)-1-cyclohexen-1-yl]ethenyl)-1,3,3-trimethylindole perchlorate (cyanin IR-786), [2-[3-[(1,3-dihydro-3,3-dimethyl-1-propyl-2H-indolyl-2-ylidenyl)ethylidenyl]-2-phenoxy-1-cyclohexen-1-yl]ethenyl]-3,3-dimethyl-1-propylindole perchlorate (cyanin IR-768), ethyl-2-[7-(3-ethyl-2-benzothiazolinylidenyl)-1,3,5-heptatrienyl]benzothiazole iodide (carbocyanin-765/ DTTCI (3,3'-diethylthiatricarbocyanins iodide)), 21H,22H-porphinyl-13,17-dipropanoic acid, 3-ethyl-8-(1-hydroksyethyl)-2,7,12,18- tetramethyl-, monosodium salt (photofrin/ Sodium porphymer) are provided as comparative examples.

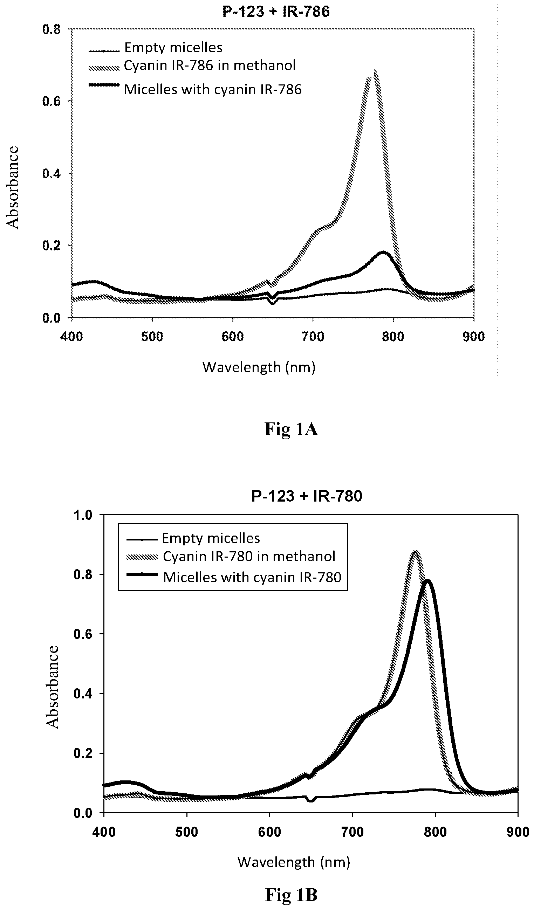

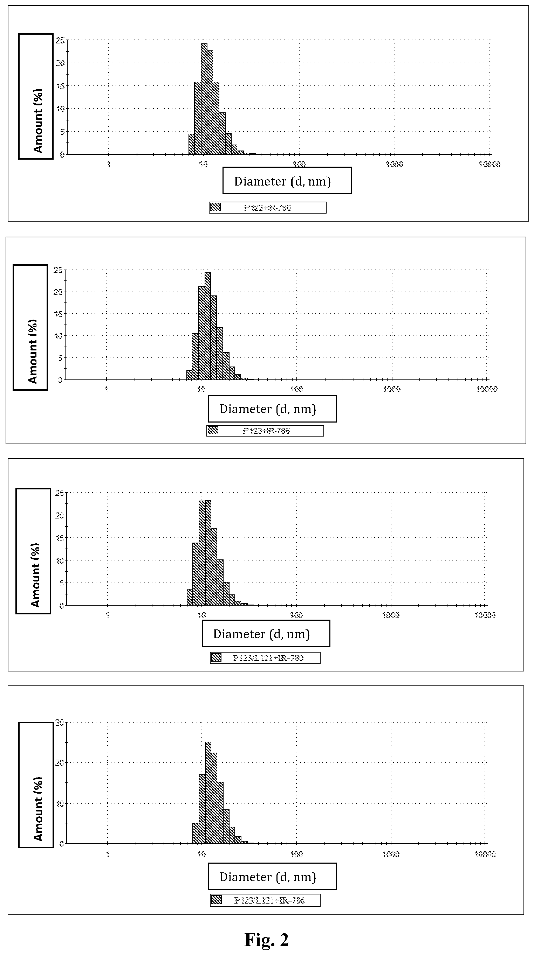

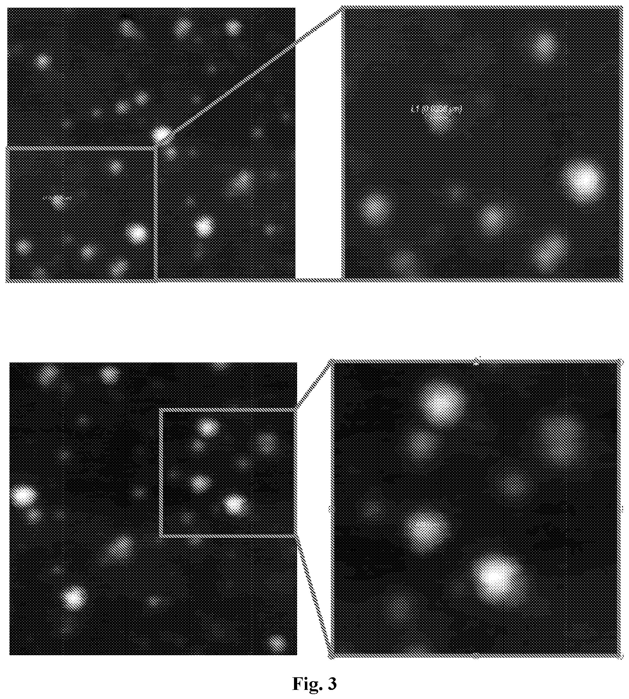

The characteristics of polymer micelles along with other parameters (hydrodynamic diameter-DH, polydispersity index-PdI, solubilized photosensitizer concentration, solubilisation degree) that describe the resulting systems are collected in Table 1, wherein systems No. 2 and 8 are embodiments of the invention and the remaining systems 1, 3-7 and 9-17 are comparative examples. Figures 1-3 show respectively: example UV-vis spectra of polymer micelles loaded with a photosensitizer in comparison to empty micelles and free cyanins dissolved in a mixture of methanol/water (1:1), results of hydrodynamic diameter measurements of polymer micelles obtained using Dynamic Light Scattering (DLS), nanocarrier morphology (example results for polymer micelles described in Example I, according to invention) obtained using Atomic Force Microscopy (AFM), wherein:
- Figure 1A relates to the comparative example P123 + IR-786, and Figure 1B to the micellar system, P123+IR-780, according to the invention;
- Figure 2 shows two systems according to the invention (P123 + IR-780, P123/L121 + IR-780) and two comparative examples (P123 + IR786, P123/L121 + IR-786).

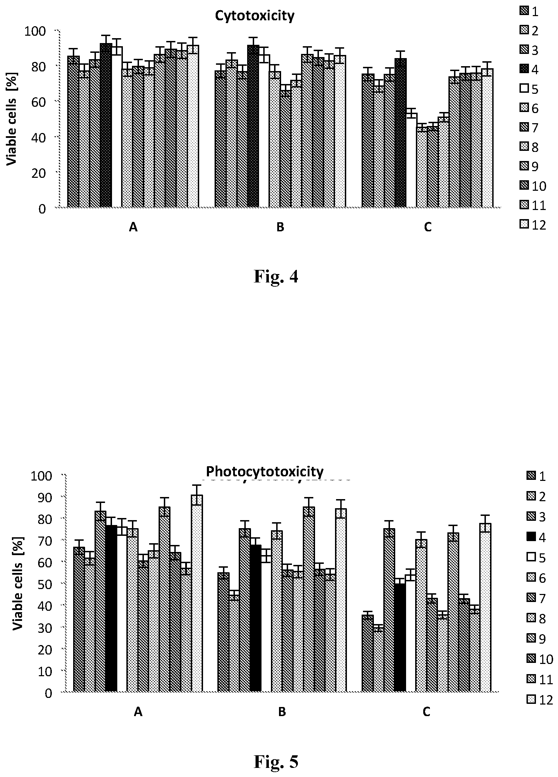

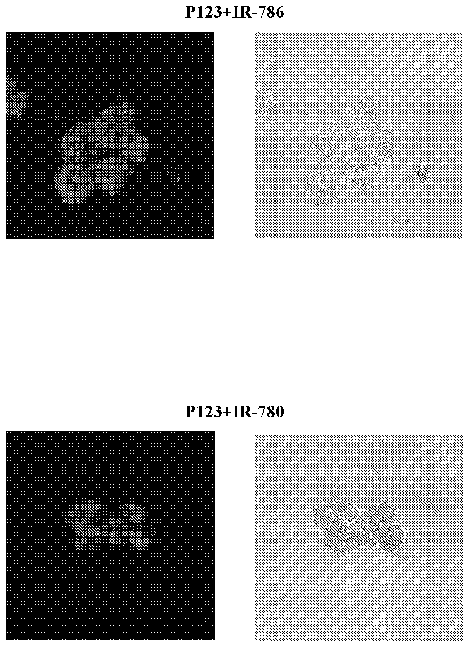

In Figures 4 and 5 (example results for systems designated in Table 1 with the numbers 1-9, wherein only systems No. 2 and 8 are according to the invention, and remaining are comparative examples) show the cytotoxic and photocytotoxic effects of a polymer micelle according to the present invention in MCF-7/WT cells in four different volumes of micelle solution added to the cells: A) 50 pL of suspension dispersed in 150 pL DMEM; B) 100 pL of suspension in 100 pL DMEM; C) 200 pL of suspension without DMEM. Sample No. 10: IR-786 4.68pM; No. 11: IR-780 4.73 pM; No. 12: blank), which, along with those loaded with photosensitizer, exhibit low cytotoxicity, whereas after illumination with a laser beam their photocytotoxicity grows in relation to free cyanins and as a result of this, they are appropriate for use in photodynamic therapy of tumours. The evaluation of cyto- and photocytotoxicity clearly demonstrates the activity of the selected photosensitizers enclosed in the polymer micelles. The results obtained show that the used carriers for photosensitizers are more effective than cyanins themselves.

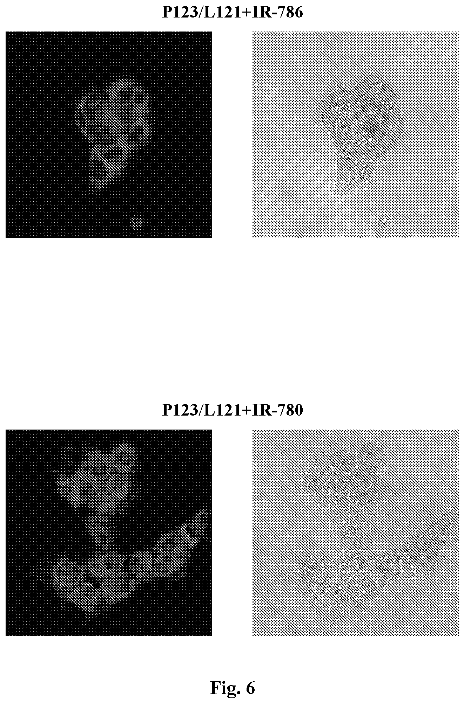

The results of research performed using FACS after incubating MCF-7WT cells (shown in Table 2) show that polymer micelles deliver the evaluated cyanins into cells much more effectively than the free drug. Intracellular localization via Confocal Laser Scanning Microscopy (CLSM) of polymer micelles loaded with photosensitizer confirms the more effective capture of the solubilised active ingredient in comparison to the free drug (Fig. 6).

### Example I (according to the invention)

10 ml of Pluronic P123 solution in methanol are mixed at a concentration of 800*CMC (CMC for Pluronic P123 is 4.4* 10-6 M) are mixed with 10ml of a solution of cyanin IR-780 in methanol with a concentration of 0.05 g/l. And then, the methanol is evaporated off on a rotating evaporator to obtain a thin film on the vessel walls and the remainder is hydrated with 10 ml water at a temperature of 50°C for 1 hour. The resulting micelle solution is filtered through a syringe filter with a pore diameter of 0.2µm in order to remove possible residues of unsolubilised photosensitizer. This results in polymer micelles whose hydrodynamic diameter D_{H} and polydispersity index (PdI) determined using dynamic light scattering (DLS) are respectively 11.9 nm and 0.058. The solubilised cyanin concentration determined by spectrophotometry is 5.19* 10⁻² g/l, whereas the degree of solubilisation is designated as the ratio of solubilised dye to total dye added is 86.5%.

### Example II (comparative example)

10 ml of Pluronic P123 solution in methanol are mixed at a concentration of 800*CMC (CMC for Pluronic P123 is 4.4* 10⁻⁶ M) are mixed with 10ml of a solution of cyanin IR-786 in methanol with a concentration of 0.05 g/l. Proceeding as in the first Example this results in polymer micelles whose hydrodynamic diameter D_{H} and polydispersity index (PdI) determined using dynamic light scattering (DLS) are respectively 12.9 nm and 0.033. The solubilised cyanin concentration determined by spectrophotometry is 2.57* 10"² g/l, whereas the degree of solubilisation is designated as the ratio of solubilised dye to total dye added is 51.4%.

### Example III (comparative example)

10 ml of Pluronic P123 solution in methanol are mixed at a concentration of 800*CMC (CMC for Pluronic P123 is 4.4* 10⁻⁶ M) are mixed with 10 ml of a solution of cyanin IR-768 in methanol with a concentration of 0.05 g/l. Proceeding as in the first Example this results in polymer micelles whose hydrodynamic diameter D_{H} and polydispersity index (PdI) determined using dynamic light scattering (DLS) are respectively 12.2 nm and 0.091. The solubilised cyanin concentration determined by spectrophotometry is 3.30* 10⁻² g/l, whereas the degree of solubilisation is designated as the ratio of solubilised dye to total dye added is 66.0%.

### Example IV (comparative example)

10 ml of Pluronic P123 solution in methanol are mixed at a concentration of 800*CMC (CMC for Pluronic P123 is 4.4* 10"⁶ M) are mixed with 10 ml of a solution of carbocyanin-765 (DTTCI) in chloroform with a concentration of 0.05 g/l. Proceeding as in the first Example this results in polymer micelles whose hydrodynamic diameter D_{H} and polydispersity index (PdI) determined using dynamic light scattering (DLS) are respectively 9.2 nm and 0.312. The solubilised cyanin concentration determined by spectrophotometry is 3.12* 10⁻² g/l, whereas the degree of solubilisation is designated as the ratio of solubilised dye to total dye added is 62.4%.

### Example V (according to the invention)

10 ml of a solution containing a mixture of Pluronic P123 and L121 in methanol with a concentration of P123 (600CMC) and L121 (200CMC) (CMC for Pluronic P-123 is 4.4* 10⁻⁶ M, whereas for Pluronic L121 1.0* 10⁻⁶ M) are mixed with 10 ml of a solution of cyanin IR-780 in methanol with a concentration of 0.05 g/l. Proceeding as in the first Example this results in polymer micelles whose hydrodynamic diameter D_{H} and polydispersity index (PdI) determined using dynamic light scattering (DLS) are respectively 12.1 nm and 0.099. The solubilised cyanin concentration determined by spectrophotometry is 4.44*10⁻² g/l, whereas the degree of solubilisation is designated as the ratio of solubilised dye to total dye added is 88.8%.

### Example VI (comparative example)

10 ml of a solution containing a mixture of Pluronic P123 and L121 in methanol with a concentration of P123 (600CMC) and L121 (200CMC) (CMC for Pluronic P123 is 4.4* 10⁻⁶ M, whereas for Pluronic L121 1.0* 10⁻⁶ M) are mixed with 10 ml of a solution of cyanin IR- 786 in methanol with a concentration of 0.05 g/l. Proceeding as in the first Example this results in polymer micelles whose hydrodynamic diameter D_{H} and polydispersity index (PdI) determined using dynamic light scattering (DLS) are respectively 13.5 nm and 0.068. The solubilised cyanin concentration determined by spectrophotometry is 3.27* 10⁻² g/l, whereas the degree of solubilisation is designated as the ratio of solubilised dye to total dye added is 65.4%.

### Example VII (comparative example)

10 ml of a solution containing a mixture of Pluronic P123 and L121 in methanol with a concentration of P123 (600CMC) and L121 (200CMC) (CMC for Pluronic P123 is 4.4x10⁻⁶ M, whereas for Pluronic L121 1.0^{*} 10⁻⁶ M) are mixed with 10 ml of a solution of cyanin IR-768 in methanol with a concentration of 0.05 g/l. Proceeding as in the first Example this results in polymer micelles whose hydrodynamic diameter D_{H} and polydispersity index (PdI) determined using dynamic light scattering (DLS) are respectively 10.7 nm and 0.068. The solubilised cyanin concentration determined by spectrophotometry is 3.01 *10⁻² g/l, whereas the degree of solubilisation is designated as the ratio of solubilised dye to total dye added is 60.2%.

### Example VIII (comparative example)

10 ml of a solution containing a mixture of Pluronic P123 and L121 in methanol with a concentration of P123 (600CMC) and L121 (200CMC) (CMC for Pluronic P-123 is 4.4* 10⁻⁶ M, whereas for Pluronic L121 1.0* 10⁻⁶ M) are mixed with 10 ml of a solution of carbocyanin-765 (DTTCI) in chloroform with a concentration of 0.05 g/l. Proceeding as in the first Example this results in polymer micelles whose hydrodynamic diameter D_{H} and polydispersity index (PdI) determined using dynamic light scattering (DLS) are respectively 11.0 nm and 0.463. The solubilised cyanin concentration determined by spectrophotometry is 1.98* 10⁻² g/l, whereas the degree of solubilisation is designated as the ratio of solubilised dye to total dye added is 39.6%.

### Example IX (comparative example)

10 ml of a solution of Pluronic L121 in methanol at a concentration of 130* CMC (CMC for Pluronic L121 is 1.0* 10⁻⁶ M) are mixed with 10 ml of a solution of cyanin IR-780 in methanol with a concentration of 0.05 g/l. Proceeding as in the first Example this results in polymer micelles whose hydrodynamic diameter D_{H} and polydispersity index (PdI) determined using dynamic light scattering (DLS) are respectively 18.3 nm and 0.358. The solubilised cyanin concentration determined by spectrophotometry is 1.29* 10⁻² g/l, whereas the degree of solubilisation is designated as the ratio of solubilised dye to total dye added is 25.8%.

### Example X (comparative example)

10 ml of a solution of Pluronic L121 in methanol at a concentration of 130* CMC (CMC for Pluronic L121 is 1.0* 10⁻⁶ M) are mixed with 10 ml of a solution of cyanin IR-768 in methanol with a concentration of 0.05 g/l. Proceeding as in the first Example this results in polymer micelles whose hydrodynamic diameter D_{H} and polydispersity index (PdI) determined using dynamic light scattering (DLS) are respectively 12.7 nm and 0.336. The solubilised cyanin concentration determined by spectrophotometry is 5.0* 10⁻³ g/l, whereas the degree of solubilisation is designated as the ratio of solubilised dye to total dye added is 10.0%.

### Example XI (comparative example)

10 ml of a solution containing a mixture of Pluronic P123 and F127 in methanol with a concentration of P123 (260CMC) and F127 (190CMC) (CMC for Pluronic P123 is 4.4* 10⁻⁶ M, whereas for Pluronic F127 2.8* 10⁻⁶ M) are mixed with 10 ml of a solution of Photofrin with a concentration of 0.16 mg/ml. And then, the methanol is evaporated off on a rotating evaporator to obtain a thin film on the vessel walls and the remainder is hydrated with 10 ml water at a temperature of 50°C for 1 hour. The resulting micelle solution is filtered through a syringe filter with a pore diameter of 0.2µm in order to remove possible residues of unsolubilised photosensitizer. This results in polymer micelles whose hydrodynamic diameter D_{H} and polydispersity index (PdI) determined using dynamic light scattering (DLS) are respectively 10,98nm and 0.212. The solubilised cyanin concentration determined by spectrophotometry is 4.51 *10⁻² g/l, whereas the degree of solubilisation is designated as the ratio of solubilised dye to total dye added is 28.2 %.

### Example XII (comparative example)

10 ml of a solution containing a mixture of Pluronic P123 and F127 in methanol with a concentration of P123 (350CMC) and F127 (130CMC) (CMC for Pluronic P123 is 4.4* 10⁻⁶ M, whereas for Pluronic F127 2.8* 10⁻⁶ M) are mixed with 10 ml of a solution of Photofrin with a concentration of 0.16 g/l. And then, the methanol is evaporated off on a rotating evaporator to obtain a thin film on the vessel walls and the remainder is hydrated with 10 ml water at a temperature of 50°C for 1 hour. The resulting micelle solution is filtered through a syringe filter with a pore diameter of 0.2µm in order to remove possible residues of unsolubilised photosensitizer. This results in polymer micelles whose hydrodynamic diameter D_{H} and polydispersity index (PdI) determined using dynamic light scattering (DLS) are respectively 13.81nm and 0.359. The solubilised cyanin concentration determined by spectrophotometry is 3.59* 10⁻² g/l, whereas the degree of solubilisation is designated as the ratio of solubilised dye to total dye added is 25.5 %.

**Table 1. Parametrs that describe the resulting systems**

| **No.** | **System** | **Copolymer concentration [mol/dm³]** | **Conc. of solubilised photosensitizer** | **Degree of solubilisation [%]** | **D_{H} [nm]** | **Pdl** |
|---|---|---|---|---|---|---|
| 1 | P P123+IR-786 | 800×CMC | 2.57×10⁻² | 51.4 | 12.9 | 0.033 |
| 2 | P P123+IR-780 | | 5.19×10⁻² | 86.5 | 11.9 | 0.058 |
| 3 | P123 empty | | - | - | 13.8 | 0.042 |
| 4 | L L121+IR-786 | 130CMC | 4.06×10⁻⁴ | 0.8 | 18.7 | 0.340 |
| 5 | L L121+IR-780 | | 1.29×10⁻² | 25.8 | 18.3 | 0.358 |
| 6 | L121 empty | | - | - | 15.5 | 0.478 |
| 7 | P1 P123/L121 +I + IR-786 | 600×CMC/ 200×CMC | 3.27×10⁻² | 65.4 | 13.5 | 0.068 |
| 8 | P123/L 121 + IR-780 | | 4.44×10⁻² | 88.8 | 12.1 | 0.099 |
| 9 | P123/L121 empty | | - | - | 17.8 | 0.476 |
| 10 | P123+IR-768 | 800×CMC | 3.30×10⁻² | 66.0 | 12.2 | 0.091 |
| 11 | P123+ Carbocyanin-765 (DTTCI) | | 3.12×10⁻² | 62.4 | 9.2 | 0.312 |
| 12 | L121+IR-768 | 130CMC | 5.0×10⁻³ | 10.0 | 12.7 | 0.336 |
| 13 | L121+ Carbocyanin -765 (DTTCI) | | 9.12×10⁻⁴ | 1.8 | 15.8 | 0.358 |
| 14 | P123/L121 +IR-768 | 600×CMC/ 200×CMC | 3.01×10⁻² | 60.2 | 10.7 | 0.068 |
| 15 | P123/L121 Carbo cyanin-765 (DTTCI) | | 1.98×10⁻² | 39.6 | 11.0 | 0.463 |
| 16 | P123/F127+ Photofrin | 260×CMC/ 190×CMC | 4.51×10⁻² | 28.16 | 1 0.98 | 0.212 |
| 17 | P123/F127 +Photofrin | 350×CMC/ 130×CMC | 3.59×10⁻² g/l | 25.53 | 1 3.81 | 0.359 |

**Table 2. FACS analysis of the capture (samples 1-9) of cyanin IR-780 and IR-786 from polymer micelles by MCF-7/WT**

| **Experimental system** | **Number of viable cells per 10 000 population** | **Percent of viable cells** | **Increase of the GMeani/ GMean0 signal** |
|---|---|---|---|
| 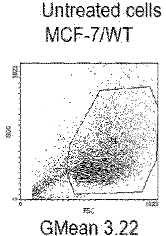 | 9005 | 90.05 | 1 |
| 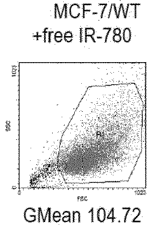 | 8928 | 89.28 | 32.5 |
| 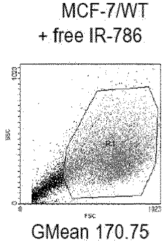 | 6857 | 68.57 | 53.02 |
| 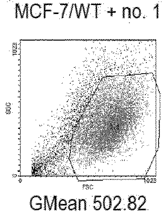 | 7525 | 75.25 | 156.2 |
| 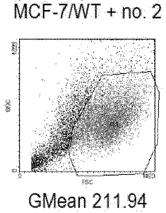 | 6234 | 62.34 | 65.82 |
| 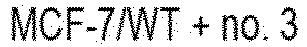 | 7971 | 79.71 | 5.68 |
| 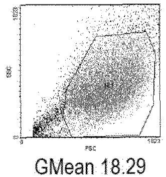 | | | |
| 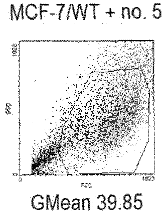 | 6946 | 69.46 | 12.38 |
| 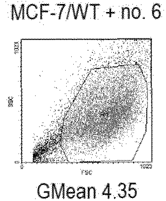 | 7539 | 75.39 | 1.35 |
| 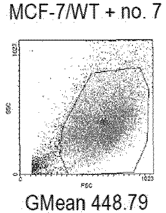 | 8254 | 82.54 | 139.4 |
| 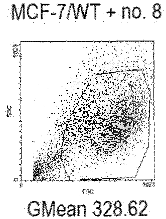 | 8136 | 81.36 | 102.06 |
| 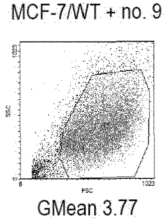 | 8226 | 82.26 | 1.2 |

## Claims

1. A polymer micelle containing an active ingredient for use in photodynamic therapy of tumours, wherein said micelle, produced using hydration method of a thin film of block copolymers, contains block copolymers: poly(ethylene oxide-6-propylene oxide-6-ethylene oxide) with the general formula 1, 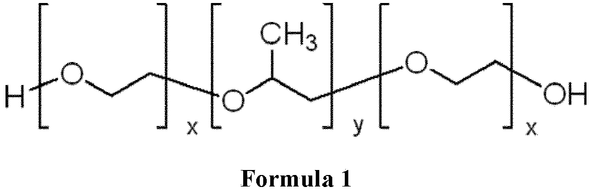 in which
x denotes the number of polyoxyethylene groups (PEO), with a value from 5 to 100;
y denotes the number of polyoxypropylene groups (PPO), with a value from 65 to 69;
wherein active ingredient is selected from a group of photosensitizer encompassing cyanins, wherein said block copolymer is a copolymer (PEO)₂₀-(PPO)₆₉-(PEO)₂₀ and said active ingredient is 2-[2-[2-chloro-3-[(1,3-dihydro-3,3-dimethyl-1-propyl-2H- indolyl-2-ylidenyl)ethylidenyl]-1-cyclohexen-1-yl]ethenyl]-3,3-dimethyl-1-propylindole iodide (cyanin IR-780).

2. A method of producing a polymer micelle defined in Claim 1, **characterised in that** block copolymers are previously dissolved in a volatile organic solvent with an active ingredient, and then the solvent is evaporated off on a rotating evaporator and the thin film formed is hydrated, wherein a solution of the copolymers is prepared with a concentration of at least a hundredfold greater than its critical micelle concentration and is mixed with a photosensitizer also dissolved in an organic solvent with a concentration of 0.01-0.2 g/l, and then the solvent is evaporated off on a rotating evaporator, resulting in thin film, which is then hydrated at a temperature of 25-60°C for 0.5-2h and an aqueous solution of micelles is obtained with the photosensitizer solubilized inside the micelle,
wherein said block copolymer is a copolymer (PEO)₂₀-(PPO)₆₉-(PEO)₂₀,
the active ingredient is 2-[2-[2-chloro-3-[(1,3-dihydro-3,3-dimethyl-1-propyl-2H- indolyl-2-ylidenyl)ethylidenyl]-1-cyclohexen-1-yl]ethenyl]-3,3-dimethyl-1-propylindole iodide (cyanin IR-780),
and the organic solvent used is selected from a group encompassing: tetrahydrofuran, methanol, ethanol, isopropanol, chloroform, preferably methanol.

3. The method according to Claim 2, **characterised in that** the photosensitizer solution is made to a concentration of 0.04-0.16 g/l.

4. The method according to Claim 2, **characterised in that** the thin layer hydration is performed at a temperature of 40-50°C, preferably for 1 hour.

5. The method according to Claim 2, **characterised in that** micelle solutions are filtered through syringe filters, preferably through filters with a pore diameter of 0.2µm.

## Patentansprüche

1. Polymermizelle, die einen Wirkstoff zur Verwendung in der photodynamischen Therapie von Tumoren enthält, wobei die Mizelle, hergestellt unter Verwendung des Hydratisierungsverfahrens eines dünnen Films von Blockcopolymeren, Blockcopolymere enthält: Poly(ethylenoxid-6-propylenoxid-6-ethylenoxid) mit der allgemeinen Formel 1, 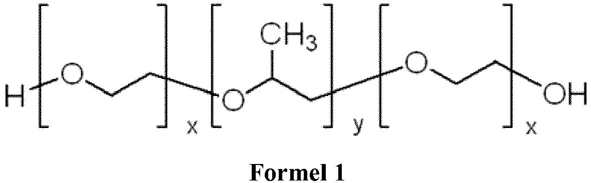 worin
x die Anzahl der Polyoxyethylengruppen (PEO) mit einem Wert von 5 bis 100 bezeichnet;
y die Anzahl der Polyoxypropylengruppen (PPO) mit einem Wert von 65 bis 69 bezeichnet;
wobei der Wirkstoff aus einer Gruppe von Photosensibilisatoren, die Cyanine umfassen, ausgewählt ist, wobei das Blockcopolymer ein Copolymer (PEO)₂₀-(PPO)₆₉-(PEO)₂₀ ist und der Wirkstoff 2-[2-[2-Chlor-3-[(1,3-dihydro-3,3-dimethyl-1-propyl-2H-indolyl-2-ylidenyl)ethylidenyl] -1 -cyclohexen-1 -yl] ethenyl] -3,3 -dimethyl-1 -propylindoliodid (Cyanin IR-780) ist.

2. Verfahren zur Herstellung einer Polymermizelle nach Anspruch 1, **dadurch gekennzeichnet, dass** Blockcopolymere zuvor in einem flüchtigen organischen Lösungsmittel mit einem Wirkstoff gelöst werden und dann das Lösungsmittel in einem Rotationsverdampfer abgedampft und der gebildete dünne Film hydratisiert wird, wobei eine Lösung der Copolymere mit einer mindestens hundertfach höheren Konzentration als ihrer kritischen Mizellenkonzentration hergestellt und mit einem Photosensibilisator gemischt wird, der ebenfalls in einem organischen Lösungsmittel mit einer Konzentration von 0,01-0,2 g/l gelöst ist, und dann das Lösungsmittel in einem Rotationsverdampfer abgedampft wird, was zu einem dünnen Film führt, der dann 0,5-2 Stunden bei einer Temperatur von 25-60°C hydratisiert wird und eine wässrige Lösung von Mizellen mit dem in der Mizelle solubilisierten Photosensibilisator erhalten wird,
wobei das Blockcopolymer ein Copolymer (PEO)₂₀-(PPO)₆₉-(PEO)₂₀ ist,
der Wirkstoff 2-[2-[2-Chlor-3-[(1,3-dihydro-3,3-dimethyl-1-propyl-2H-indolyl-2-ylidenyl)ethylidenyl] -1 -cyclohexen-1 -yl] ethenyl] -3,3 -dimethyl-1 -propylindoliodid (Cyanin IR-780) ist,
und das verwendete organische Lösungsmittel aus einer Gruppe umfassend: Tetrahydrofuran, Methanol, Ethanol, Isopropanol, Chloroform, vorzugsweise Methanol ausgewählt ist.

3. Verfahren nach Anspruch 2, **dadurch gekennzeichnet, dass** die Photosensibilisatorlösung auf eine Konzentration von 0,04-0,16 g/l hergestellt wird.

4. Verfahren nach Anspruch 2, **dadurch gekennzeichnet, dass** die Hydratation der Dünnschicht bei einer Temperatur von 40-50°C, vorzugsweise für 1 Stunde, durchgeführt wird.

5. Verfahren nach Anspruch 2, **dadurch gekennzeichnet, dass** Mizellösungen durch Spritzenfilter, vorzugsweise durch Filter mit einem Porendurchmesser von 0,2 µm, gefiltert werden.

## Revendications

1. Micelle de polymère contenant un principe actif destiné à être utilisé dans la thérapie photodynamique des tumeurs, ladite micelle étant produite en utilisant un procédé d'hydratation d'un film mince de copolymères à blocs et contient des copolymères à blocs : poly(oxyde d'éthylène-6-oxyde de propylène-6-oxyde d'éthylène) ayant une formule générale 1, 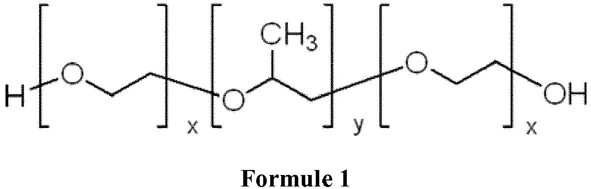 où
x représente le nombre de groupes polyoxyéthylène (PEO), ayant une valeur de 5 à 100;
y représente le nombre de groupes polyoxypropylène (PPO), ayant une valeur de 65 à 69 ;
dans lequel l'ingrédient actif est choisi dans un groupe de photosensibilisants comprenant des cyanines, dans lequel ledit copolymère à blocs est un copolymère (PEO)₂₀-(PPO)₆₉-(PEO)₂₀, et ledit ingrédient actif est le 2-[2-[2-chloro-3-[(1,3-dihydro-3,3-diméthyl-1-propyl-2H-indolyl-2-ylidényl)éthylidényl]-1-cyclohexène-1-yl] éthényl]-3,3-diméthyl-1-propylindole (cyanine IR-780).

2. Procédé de production d'une micelle de polymère définie dans la revendication 1, **caractérisé en ce que** les copolymères à blocs sont préalablement dissous dans un solvant organique volatil avec un principe actif, puis le solvant est évaporé sur un évaporateur rotatif et le film mince formé est hydraté, dans lequel une solution des copolymères est préparée avec une concentration au moins cent fois supérieure à sa concentration micellaire critique et est mélangée à un photosensibilisant également dissous dans un solvant organique à une concentration de 0,01 à 0,2 g/l, puis le solvant est évaporé sur un évaporateur rotatif, ce qui donne un film mince, qui est ensuite hydraté à une température de 25 à 60°C pendant 0,5 à 2 heures et l'on obtient une solution aqueuse de micelles avec le photosensibilisant solubilisé à l'intérieur de la micelle,
dans lequel ledit copolymère à blocs est un copolymère (PEO)₂₀-(PPO)₆₉-(PEO)₂₀,
l'ingrédient actif est 2-[2-[2-chloro-3-[(1,3-dihydro-3,3-diméthyl-1-propyl-2H-indolyl-2-ylidényl)éthylidényl] -1-cyclohexène-l-yl] éthényl] -3,3 -diméthyl-1-propylindole iodure (cyanine IR-780),
et le solvant organique utilisé est choisi dans un groupe comprenant: tétrahydrofuranne, méthanol, éthanol, isopropanol, chloroforme, de préférence méthanol.

3. Procédé selon la revendication 2, **caractérisé en ce que** la solution de photosensibilisant est préparée à une concentration de 0,04-0,16 g/l.

4. Procédé selon la revendication 2, **caractérisé en ce que** l'hydratation en couche mince est effectuée à une température de 40 à 50°C, de préférence pendant 1 heure.

5. Procédé selon la revendication 2, **caractérisé en ce que** les solutions de micelles sont filtrées à travers des filtres à seringue, de préférence à travers des filtres ayant un diamètre de pores de 0,2 µm.
